# EUROPEAN PATENT SPECIFICATION

(11) **EP 1 194 132 B1**
(45) Date of publication and mention of the grant of the patent: **16.06.2004**
(21) Application number: 00954518.7
(22) Date of filing: 27.06.2000
(51) Int. Cl.: A61K 9/50, A61K 9/00

(54) **TIMED DUAL RELEASE DOSAGE FORMS COMPRISING A SHORT ACTING HYPNOTIC OR A SALT THEREOF**
ZEITGESTEUERT FREISETZENDE DOSIERUNGSFORMEN ENTHALTEND EIN KURZ WIRKSAMES HYPNOTIKUM ODER EIN SALZ DAVON
FORMES DE DOSAGE A DOUBLE LIBERATION DANS LE TEMPS COMPRENANT UN HYPNOTIQUE, OU UN DE SES SELS, A ACTION BREVE

(30) Priority: 28.06.1999 EP 99401605
(43) Date of publication of application: 10.04.2002
(73) Proprietor: SANOFI-SYNTHELABO, 75013 Paris (FR)
(72) Inventor: ALAUX, Gérard, F-78650 Beynes (FR); ANDRE, Frédéric, F-92160 Antony (FR); DUCASSOU, Jean, F-64600 Anglet (FR); LEWIS, Gareth, F-91410 Dourdan (FR)
(74) Representative: Thouret-Lemaitre, Elisabeth
(86) International application number: PCT/EP2000/006792
(87) International publication number: WO 2001/000181

(56) References cited:
- EP-A- 0 908 177
- WO-A-96/41617
- WO-A-97/23219
- FR-A- 2 656 303
- GB-A- 2 185 887

## Description

The present invention relates to timed dual release dosage forms comprising short acting hypnotics or salts thereof.

The short acting hypnotics can belong to all therapeutic classes:
- compounds of the therapeutic class of pyrazolopyrimidines, such as zaleplon,
- compounds of the therapeutic class of cyclopyrrolones, such as zopiclone and its enantiomers like (*R*)-zopiclone,
- compounds of the therapeutic class of benzodiazepines, such as triazolam, temazepam or brotizolam,
- compounds of the therapeutic class of phenothiazines, such as alimemazine or the tartrate thereof,
- compounds of the therapeutic class of imidazopyridines, such as zolpidem.
One of the preferred salts of zolpidem is zolpidem hemitartrate.

Up to now, according to the rapidity of action of this kind of active substances, only immediate release dosage forms were developed, which disintegrate rapidly in the gastro-intestinal tract, dissolve in the fluid of the gastrointestinal tract and undergo systemic absorption, where the short acting hypnotic, which we will call hereinafter "the drug", can exert its pharmacological effect and induce sleep of the patient.

WO-A-96/41617 relates to a combination of an immediate release and a sustained (slow) release of active ingredients which are required to exhibit not only an immediate therapeutic effect but also the maintenance of activity over a more prolonged period.

EP-A-0 908 177 discloses a combination of an immediate release and a sustained (slow) release of bromazepam, in the form of a composition of substantially spherical granules or pills of controlled and sustained action, capable to form stable plasmatic concentrations and effective levels of bromazepam during 24 hours.

The new dosage forms according to the present invention enable first of all a sufficient blood level of short acting hypnotic to be obtained rapidly after administration in order to induce sleep, and then a second pulse of short acting hypnotic to be released after a fixed time after administration in order to maintain sleep.

Therefore, as a first object, the present invention provides timed dual release dosage forms comprising short acting hypnotics or salts thereof adapted to release over a predetermined time period, according to a profile of dissolution, characterized in that it comprises two release pulses, the first being immediate and the second being delayed to a fixed time.

The "total amount of drug" means the quantity by weight of the drug comprised in the whole dosage form according to the invention.

The immediate release portion of the profile (initial pulse) is defined as that proportion of the drug dissolved in 30 minutes in a suitable *in vitro* dissolution test. A suitable dissolution test is for example one of the methods described in example 1: method where measurement is carried out with the rotating paddle apparatus of the European pharmacopoeia, at a stirring speed of 50 rpm, in an aqueous buffer at pH between 1 and 7.5 at 37°C, or variations on this as well known to one skilled in the art.
The proportion of the drug dissolved during this pulse is the proportion of the total amount of the drug which is dissolved at 30 minutes. In an advantageous embodiment of the dosage forms according to the present invention 90% or more of that part of the drug allotted for the initial pulse is dissolved in 20 minutes and more preferably in 15 minutes. This embodiment is particularly advantageous for dosage forms comprising zolpidem or a salt thereof.

The delayed release portion of the profile is the part of the dissolution occurring after 30 minutes, measured in a suitable *in vitro* dissolution test, such as described in example 1.
The delayed release portion of the profile is defined by the percentage released at times T₁ and T₂, defined as follows.
T₁ describes the beginning of the second, delayed release pulse, and is defined as the time for release of 10% of the drug allotted for the delayed release portion of the profile.
T₂ describes the end of the delayed release pulse, and is defined as the time for release of 85% of the drug allotted for the delayed release portion of the profile.

The release of the delayed release pulse may be less rapid than the immediate release pulse. For example, the period (T₂-T₁) can last between 30 and 200 minutes.

Moreover, the delayed release pulse can begin between 50 minutes and 200 minutes after the beginning of dissolution, and preferably between 60 and 150 minutes, this range of time being defined as the "fixed time".

Indeed, the delayed release should be completed at a time after administration compatible with the desired time of sleep, and the time needed for elimination of the drug from the human body to a sufficiently low level roughly 8 hours after administration. In view of this, T₂ is between 2 and 6 hours and preferably between 2.5 and 5 hours.

The immediate release pulse can liberate between 40 to 70% of the total amount of the drug.

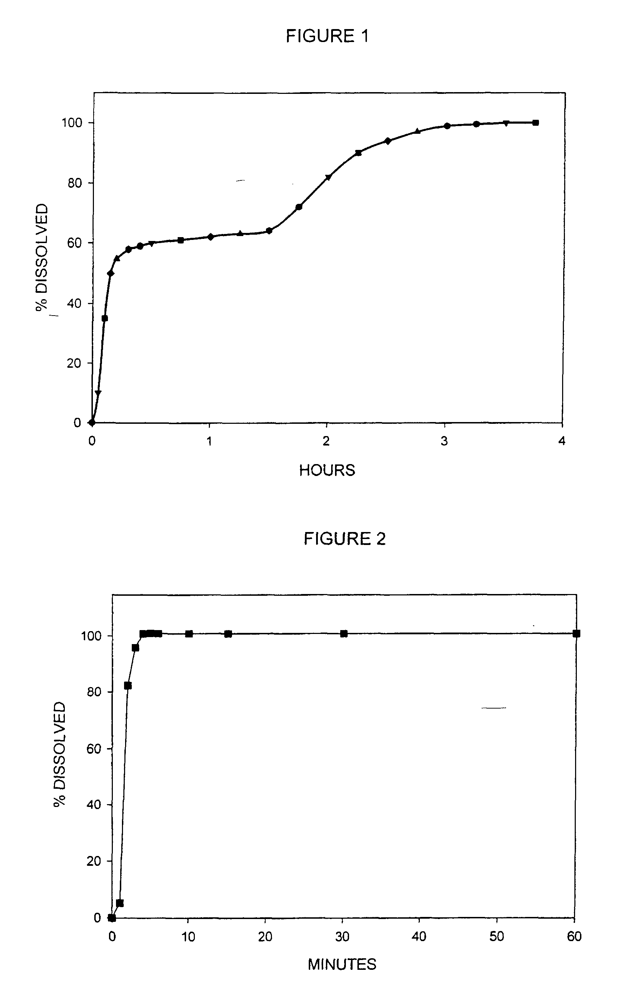

An example of such an *in vitro* release profile is given in figure 1, where 60% of the total amount of drug is released during the immediate release pulse, and the second, delayed release pulse occurs after 90 minutes (T₁) with a T₂ time being equal to 150 minutes.

As a second object, the present invention provides timed dual release dosage forms of short acting hypnotics or salts thereof, characterized in that they comprise two kinds of pharmaceutical entities of drug: one immediate release entity and one delayed release entity. The drug dissolved during the initial, immediate release pulse (before 30 minutes) is contained within the immediate release entity, and that liberated in the second, delayed release pulse (beginning after the fixed time) is contained within the delayed release entity.

Small quantities of the drug in a formulation for rapid release can be retained in the formulation and thus may be released at a time after 30 minutes from the beginning of the dissolution, and are thus included in the delayed release part of the profile. Similarly, small quantities of the drug incorporated in the delayed release pharmaceutical entity may be released before 30 minutes, and thus form part of the immediate release part of the profile.
According to the present invention, the proportion of the drug contained within the immediate release entity and dissolved within 30 minutes is at least 90%. And the proportion of the drug contained within the delayed release entity and released within 30 minutes is comprised between 0 and 20%, and preferably between 0 and 5%.

Among dosage forms able to match the requirement of a timed dual release profile and to comprise the two kinds of pharmaceutical entities defined above, the following may be cited: capsules, tablets, multilayer tablets, multicoated tablets.

The immediate release entity shall be understood in the present invention as a single pharmaceutical immediate release unit like for example an immediate release tablet or pellet, or several such units formulated into a capsule or a tablet ; as an immediate release matrix in a tablet ; as an immediate release layer, that can be incorporated in a multilayer tablet ; as an immediate release coating layer in a multicoated tablet or pellet.

The delayed release entity shall be understood in the present invention as a pharmaceutical delayed release unit such as, for example, a delayed release tablet or pellet, or several such units formulated into a capsule or a tablet ; as a delayed release core or a delayed release coating layer in a multicoated tablet ; as delayed release pellets within a disintegrating tablet.

Dosage forms where the immediate release entity and the delayed release entity are administered simultaneously but separately are also encompassed in the present invention.

The total amount of short acting hypnotic contained in the dosage forms according to the present invention depends on the individual drug.
For example, the dosage forms according to the invention typically contain from 10 to 30 mg of zaleplon or from 7.0 to 15 mg of zopiclone.
In the same way, the dosage forms according to the invention typically contain from 4 to 16 mg of zolpidem as zolpidem base, and preferably 6 to 12 mg of zolpidem as zolpidem base. The zolpidem may be incorporated as the base, or as a pharmaceutically acceptable salt of zolpidem. Among dosage forms comprising a salt of zolpidem rather than the zolpidem base, according to the invention, those comprising zolpidem hemitartrate are especially preferred.

In advantageous embodiments, dosage forms may be formulated in order to obtain a dissolution independent of the pH in the second release pulse. The preferred _manner to achieve such a dissolution in the case of a basic short acting hypnotic, like zolpidem, zopiclone or zaleplon, is to add a pharmaceutically acceptable organic acid into the dosage form, according to methods known from one skilled in the art. Such dosage forms are preferred.
These pharmaceutically acceptable organic acids can be chosen for example among maleic, tartaric, malic, fumaric, lactic, citric, adipic or succinic acid and their acid salts where these exist, in the form of racemates or isomers, where these exist. According to the invention, acids particularly preferred are tartaric, fumaric, citric, and succinic and their acid salts.

Various formulations, not limiting the scope of the present invention, illustrating the invention are described hereafter:
(1) A mixture of immediate release and delayed release particles of dimension 0.2 - 2 mm, known variously as pellets, beads, granules or spheroids, within a capsule:
   The beads, pellets, granules or spheroids may be manufactured by any of the methods well known to one skilled in the art: granulation in a high speed granulator, extrusion followed by spheronisation, gradual coating of a sugar sphere consisting of sugar or microcrystalline cellulose or mannitol or any other suitable pharmacologically inert substance, with a mixture containing the drug etc.
   A part of the pellets, granules or spheroids is then coated for delayed release as described hereinafter. The coating must be impermeable to the drug on contact with aqueous fluid, but becomes permeable to the drug after a suitable period as described above, and not earlier, as a result either of erosion of the coating, or by increase in permeability of the coating, for example by the formation of aqueous pores, or by breakdown of the film, which may be obtained by means of:
   (i) a coating which contains one or more polymers impermeable to water and to drug molecules, such as ethylcellulose, ammonio methacrylate copolymer Type B, cellulose acetate, cellulose acetate butyrate, polyvinyl chloride, polyvinylacetate and one or more polymers which are permeable to water, such as hydroxypropylmethyl-cellulose, hydroxyethylcellulose, methylcellulose ammonio methacrylate copolymer Type A , the composition of the mixture being adjusted to allow gradual hydration of the film and a delayed release dissolution profile.
   (ii) a coating containing a mixture of polymers as in (i) which are physically incompatible with one another (immiscible). An example of such a mixture is that of ethylcellulose and methacrylate copolymers with quaternary ammonium groups (ammonio methacrylate copolymer Type A or B).
   (iii) a hydrophobic erodible coating, consisting of a wax such as carnauba wax, glyceryl behenate, or hydrogenated castor oil. This may be mixed with one or more insoluble diluants such as calcium dihydrogen phosphate or talc. It can be applied as the melted wax, for example in a fluid-bed coating apparatus.

   According to a preferred embodiment, in the case where one or more of the coating polymers is an ammonio methacrylate copolymer, a suitable cationic surfactant, or an amphoteric or zwitterionic surfactant is added into the core.
   The surfactant diffuses into the coating, and at a given level provokes a sudden change in the films properties, provoking a sudden rapid release.
   This particular embodiment presents the advantage that the delayed pulse is accelerated and gives substantially more complete release of the active substance than for pellets, granules or spheroids coated with methacrylate copolymer without surfactants in their cores.
   Examples of such cationic surfactants are trimethyl-dimyristoyl-ammonium propane, dimethyl-dioctadecyl-ammonium bromide, trimethyl-cetyl-ammonium bromide (CTAB), dimethyl-didodecyl-ammonium bromide (DDAB(12)), benzalkonium chloride, cetylpyridinium chloride, cetramide.
   Examples of zwitterionic surfactants are the N-alkybetaines, the C-alkylbetaines, the N-alkylamidobetaines such as cocamidopropylbetain, the N-alkylglycines and the phosphatidylcholines or lecithines.
   This method is employed in the case of examples 2, 3 and 5.
   The said preferred formulation where one or more of the coating polymers is an ammonio methacrylate copolymer can also contain a mixture of cationic and/or zwitterionic surfactants especially mixtures of the afore mentioned surfactants.
   In the case of the coating being a hydrophobic wax, one or more non-ionic surfactants may be included in the formulation, in the core, in order to promote dissolution and erosion of the film.
   The core may contain other substances known to be necessary or advantageous to formation by one skilled in the art of pharmaceutical formulation, in particular an organic acid to maintain the pH at the interior of the pellet constant. The core may also be coated with a water-soluble polymer, for example hydroxypropypylmethylcellulose or polyvinylpyrrolidone, before application of the outer coating, in order to eliminate contact between the core and the outer coating.
(2) A mixture of delayed release particles and an immediate release powder, within a capsule:
   The delayed release particles, known variously as granulates, pellets, beads, microspheres are those described above in (1). The immediate release powder is prepared by a simple mixture of the drug with pharmaceutically inactive substances, or by granulation of a mixture of drug mixed with pharmaceutically inactive substances, using one of the granulation methods well known to one skilled in the art of pharmaceutical formulation.
(3) A tablet containing delayed release coated pellets as described in (1) containing the drug imbedded in a matrix also containing the drug.
   Alternatively the tablet may consist of a mixture of delayed release coated pellets and of immediate release non-coated pellets containing the drug, imbedded in a matrix free from the drug.
   Alternatively the delayed release coated pellets may be furthermore coated with a layer containing the drug and other excipients allowing immediate release from that layer, imbedded in a matrix free from the drug.
   Alternatively the tablet may consist of one or more layers containing delayed release coated pellets containing the drug, imbedded in a matrix free from the drug and one or more layers containing the drug in an immediate release matrix.
   The matrix surrounding the pellets should preferably be formulated so that the compression into tablets does not interfere with the integrity of the membrane surrounding the pellets. On contact with fluid the tablet disintegrates, releasing the drug rapidly, from the matrix, or the immediate release pellets, or from the immediate release pellet coating, or from the immediate release layer, and then, after a set interval of time, releasing the drug from the delayed release pellets The pellet may be formulated with a pharmaceutically acceptable organic acid so as to maintain the micro-pH of the pellet during dissolution in the neutral pH conditions. The matrix consists of inert pharmaceutical substances such as well known to one skilled in the art of pharmaceutical formulation. In particular the matrix includes one or more diluants such as microcrystalline cellulose, lactose, mannitol, starch and one or more disintegrants, for example crospovidone, sodium starch glycolate and croscarmellose. Other excipients may also be included, lubricants, for example magnesium stearate, glyceryl stearate, and glyceryl behenate, binders, for example hydroxypropylmethyl-cellulose, ethylcellulose and povidone, glidants, for example talc and colloidal silicon dioxide.
(4) A capsule containing one or more immediate release tablets and one or more delayed release tablets.
   The immediate release tablet or tablets may be formulated by the methods well known to one skilled in the art. In addition to the drug they can contain inert pharmaceutical excipients, including one or more diluants, for example microcrystalline cellulose, lactose, mannitol, starch ; and may contain other excipients. These can include one or more binders, for example hydroxypropylmethylcellulose, ethylcellulose and povidone, lubricants, for example magnesium stearate, glyceryl stearate, and glyceryl behenate, disintegrants, for example crospovidone, sodium starch glycolate and croscarmellose, glidants, for example talc and colloidal silicon dioxide.

The cores of the delayed release tablets may be prepared using the same excipients as the immediate release tablets except that additional substances may be added. In particular a pharmaceutically acceptable acid may be added to ensure liberation of the drug independent of the pH of the external medium.

The delayed release tablets are coated with a layer of polymer coating similar to those described for the multiparticulate pellet systems above. However some modification of the coating will be required because of the difference in surface area of the dosage form. It is usually necessary to apply a thicker coating on the tablet than on the pellets, and thus a higher proportion of water-permeable polymers will be required in the coating composition.
In the case of a hydrophobic wax coating, the wax may be mixed with a soluble diluant such as polyethylene glycol, and the mixture applied by press coating.
In the case of coatings containing a methacrylate copolymer, a cationic surfactant may advantageously be included within the delayed release tablet core.
In the case of coatings containing a hydrophobic, waxy excipient such as carnauba wax or hydrogenated castor oil, a non-ionic surfactant may be included within the tablet core. The tablet core and the coating may also be separated by a coating of a water-soluble polymer, for example hydroxypropypylmethylcellulose or polyvinylpyrrolidone.

As other particular embodiments encompassed within the scope of the present invention, pharmaceutical compositions intended to avoid abuse may be cited.
Indeed it is known that some drugs and in particular hypnotics intended for legitimate oral use have the potential for abuse.
One way of substantially reducing or even eliminating this potential for drug abuse for the pharmaceutical formulations that are objects of the present invention is to provide pharmaceutical compositions for oral administration comprising a short acting hypnotic or a salt thereof capable at the same time of:
- liberating the active principle according to a timed dual release *in vitro* profile as described above, following normal administration and,
- if it is introduced in a drink, whether or not containing alcohol, generating visual change or changes in the appearence of the drink. This visual change or changes are intended to avoid administration of the active principle to a person in the said drink without his or her knowledge.

These visual changes, according to the present invention include all means of indicating the presence of the said composition in a drink. The following may be cited as methods for inducing visual changes: inclusion of colouring excipients, floating of the composition at the surface of the drink, formation of insoluble particles on the surface of the drink, on the brim of the glass, in the drink and/or on the bottom of the glass or a combination thereof.
The drink, eventually with alcohol, may for example consist of coffee, tea, wine, fortified wine, spirits, liqueurs, hot or cold chocolate-flavoured drinks, all gaseous alcoholic or not-alcoholic drinks, all cocktails or mixtures of fruit juice, milk, cream, ...

Particles may be obtained by association of a lipophilic and a hydrophilic excipient, useful for the floating as described above. A list of suitable lipophilic excipients is set forth beneath.

The composition according to this particular embodiment of the present invention can liberate particules even if the composition does not float or not immediately.

Among lipophilic excipients the following may be cited: glycerol stearates, palmitostearates and behenates; hydrogenated vegetable oils and their derivatives; vegetable and animal wax and their derivatives; hydrogenated castor oils and their derivatives and cetylic esters and alcohols.
Among hydrophilic excipients the following may be cited: cellulose derivatives, hydroxyethylcellulose, hydroxypropylcellulose (molecular mass from 50 to 1250 kDa), hydroxypropylmethylcellulose (molecular mass from 10 to 1500 kDa), carboxymethylcellulose and sodium carboxymethylcellulose; vegetable gums and their derivatives; derivatives of alginic acid; polyethyleneglycols and their derivatives; starches and their derivatives; silica, polymethacrylates and acrylic acid and methacrylate copoplymers.
One of the constituants of the gel forming substance can be chosen as being less soluble in alcohol.

A colouring excipient can be advantageously added as giving rise to visual change preventing abuse. It can colour simultaneously the liquid or the particles or one independantly of the other.
Among suitable colouring excipients the following may be cited : indigotine, cochineal carminic acid, yellow orange S, allura red AC, iron oxides, cucurmin, riboflavin, tartrazine, quinoline yellow, azorubine, amaranth, carmines, erythosine, red 2G, patented blue V, glittering blue FCF, chlorophylls, copper complexes of chlorophylls, green S, caramel, glittering black BN, carbo medicinalis vegetabilis, brown FK and HT, carotenoids, Annatto extracts, paprika extracts, lycopene, lutein, canthaxanthin, beetroot red, anthocyanes, calcium carbonate, titanium dioxide, aluminium, silver, gold or litholrubin BK or any other colouring excipient suitable for an oral administration.

These visual means of preventing abuse may comprise a distinct pharmaceutical entity, not containing active substance, along with the immediate release and the sustained release entities, that comprise the pharmaceutical form, or they may be incorporated in one of these two entities. Yet a third method is to incorporate all or certain of them into a separate entity and at the same time add certain to the immediate or sustained release entity.
The method of incorporation of abuse resistance as described above will depend on the type of formulation. In the case of tablet formulations described above, including that of tablets enclosed inside a capsule, the abuse resistance conferring substances (colouring matter, effervescent couple...) may be included within the immediate release entity of the formulation.
Alternatively in the case of multilayer tablets and immediate tablets within a capsule they may be incorporated as a separate layer not containing active substance, but with the abuse resistance conferring substances. Such a layer may be added to the sustained release tablet or tablets within a capsule provided the said tablet is formulated as a matrix and is not coated with a coating conferring the sustained release properties.
In the case of a capsule containing controlled release pellets and immediate release pellets or granulate, abuse resistance conferring substances may be incorporated in the immediate release entity or added separately.

### List of figures:

Figure 1 shows an example of a *in vitro* timed dual release profile, where the immediate release pulse is 60% of the total amount of zolpidem, and the second pulse is 40%, starting at 90 minutes and finishing at 150 minutes.
Figure 2 shows an *in vitro* dissolution profile of the uncoated pellets containing zolpidem hemitartrate of example 1 at pH 2.
Figure 3 shows an *in vitro* dissolution profile of the coated pellets containing zolpidem hemitartrate of example 2 at pH 2 and 6.8.
Figure 4 shows an *in vitro* dissolution profile of the coated pellets containing zolpidem hemitartrate of example 3 at pH 2 and pH 6.8.
Figure 5 shows the *in vitro* dissolution profile of a capsule of example 4 containing a mixture of the uncoated pellets of example 1 and the coated delayed release pellets of example 3, containing 7.5 mg zolpidem hemitartrate in each type of pellet, at pH 2.
Figure 6 shows *in vitro* release profiles of coated pellets containing zolpidem hemitartrate of comparative example 1 at pH 2 and 6.8.
Figure 7 shows *in vitro* release profiles of coated pellets containing zolpidem hemitartrate of comparative example 2 at pH 2 and 6.8.
Figure 8 shows the *in vitro* dissolution profile of the coated pellets containing zolpidem tartrate of example 5.
The examples which follow illustrate the invention without limiting it:

### Example 1: Immediate release pellets containing zolpidem hemitartrate

1000 g of nonpareil beads 16/18 mesh were coated using a suspension with the following composition,

| | | |
|---|---|---|
| zolpidem hemitartrate | 11.54 % | 78.125 g |
| povidone K30 ¹ | 11.54 % | 78.125 g |
| Ethanol | 76.92 % | 520.8 g |

| | | |
|---|---|---|
| ¹ Kollidon® commercialised by BASF | | |

The coating was carried out using a GPCG1 fluid bed coated-dryer (Glatt). The dissolution of the beads was measured using the method described in the European pharmacopoeia, with the rotating paddle apparatus, at a stirring speed of 50 rpm. Dissolution medium was 900 ml, 0.01M hydrochloric acid, at 37 ± 0.5°C. The amount of zolpidem hemitartrate dissolved was measured by UV spectrophotometry at 310 nm. The dissolution curve obtained is shown in figure 2.

### Example 2: Coated pellets:

Delayed release pellets containing zolpidem hemitartrate, tartaric acid and benzalkonium chloride as cationic surfactant
1000 g of nonpareil beads 16/18 mesh were coated using a suspension with the following composition,

| | | |
|---|---|---|
| tartaric acid | 6.0 % | 78 g |
| hydroxypropylmethylcellulose ¹ | 4.0 % | 53 g |
| benzalkonium chloride | 3.0 % | 39 g |
| purified water | 43.5 % | 567 g |
| isopropanol | 43.5 % | 567 g |

| | | |
|---|---|---|
| ¹ Pharmacoat® 603 commercialised by Shin-Etsu | | |

The pellets were then loaded with zolpidem hemitartrate by coating with the following solution, in a GPCG1 fluid bed coater-dryer:

| | | |
|---|---|---|
| zolpidem hemitartrate | 8.3 % | 78 g |
| povidone K30 ² | 8.3 % | 78 g |
| ethanol | 83.4 % | 784 g |

| | | |
|---|---|---|
| ² Kollidon® commercialised by BASF | | |

Finally the pellets were coated using a polymer solution of the following composition :

| | | | |
|---|---|---|---|
| ammonio copolymer Type B ³ | methacrylate | 11.40 % | 83.4 g |
| ammonio copolymer Type A ⁴ | methacrylate | 0.93 % | 6.8 g |
| triethyl citrate ⁵ | | 1.37 % | 10.0 g |
| isopropanol | | 51.80 % | 379.0 g |
| acetone | | 34.50 % | 252.0 g |

| | | | |
|---|---|---|---|
| ³ Eudragit® RS100 commercialised by Röhm Pharma | | | |
| ⁴ Eudragit® RL100 commercialised by Röhm Pharma | | | |
| ⁵ Eudraflex® commercialised by Röhm Pharma | | | |

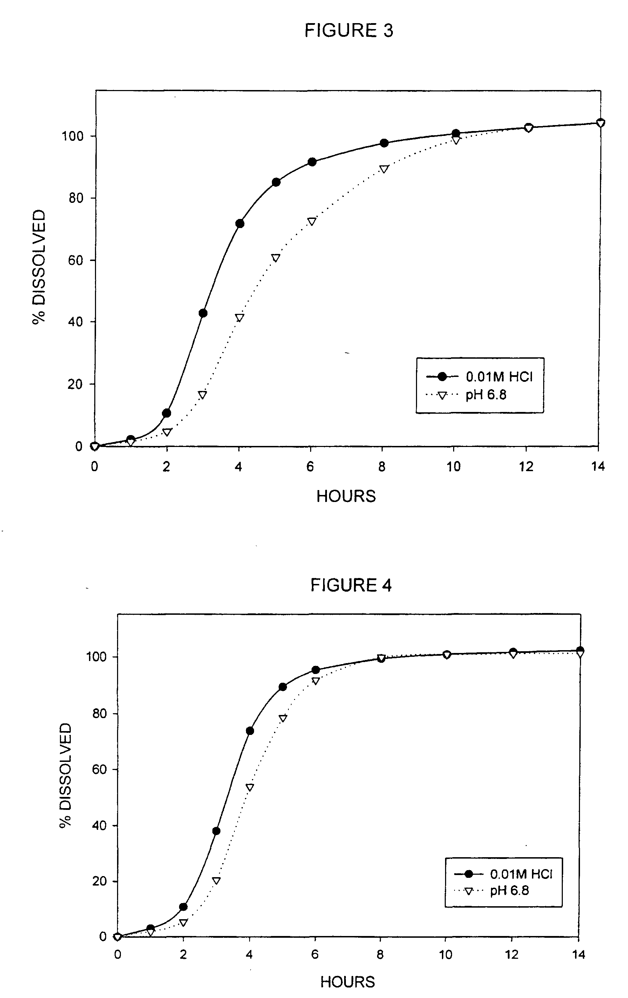

The dissolution profiles of the pellets were tested in 0.01M hydrochloric acid using the method described in example 1, and in a 0.02M pH 6.8 potassium phosphate buffer solution containing 0.1M sodium chloride, all other parameters being the same as for the test in hydrochloric acid. They are shown in figure 3.

### Example 3: Coated pellets:

Delayed release pellets containing zolpidem hemitartrate, tartaric acid and cetylpyridinium chloride as cationic surfactant
1000 g of nonpareil beads 16/18 mesh were coated using a suspension with the following composition:

| | | |
|---|---|---|
| tartaric acid | 6.0 % | 78.0 g |
| hydroxypropylmethylcellulose ¹ | 4.0 % | 53.0 g |
| cetylpyridinium chloride | 3.0 % | 39.0 g |
| triethyl citrate ² | 1.4 % | 18.2 g |
| purified water | 42.8 % | 557.0 g |
| isopropanol | 42.8 % | 557.0 g |

| | | |
|---|---|---|
| ¹ Pharmacoat® 603 commercialisé par Shin-Etsu | | |
| ² Eudraflex® commercialized by Röhm Pharma | | |

The beads were then loaded with zolpidem hemitartrate by coating, in a GPCG1 fluid bed coated-dryer, and finally coated using a polymer solution, using the same methods and compositions as described in example 2. The dissolution profiles of the pellets were measured as described in example 2. These are shown in figure 4.

### Example 4: capsule containing a mixture of immediate release and delayed release pellets containing zolpidem hemitartrate

Capsules containing 15 mg zolpidem hemitartrate were manufactured according to the following composition :

| Component | Mass per unit | Zolpidem hemitartrate content |
|---|---|---|
| uncoated beads of example 1 | 112 mg | 7.5 mg |
| coated beads of example 3 | 131 mg | 7.5 mg |
| hard gelatine capsule size 3 | | - |
| (Total) | | 15.0 mg |

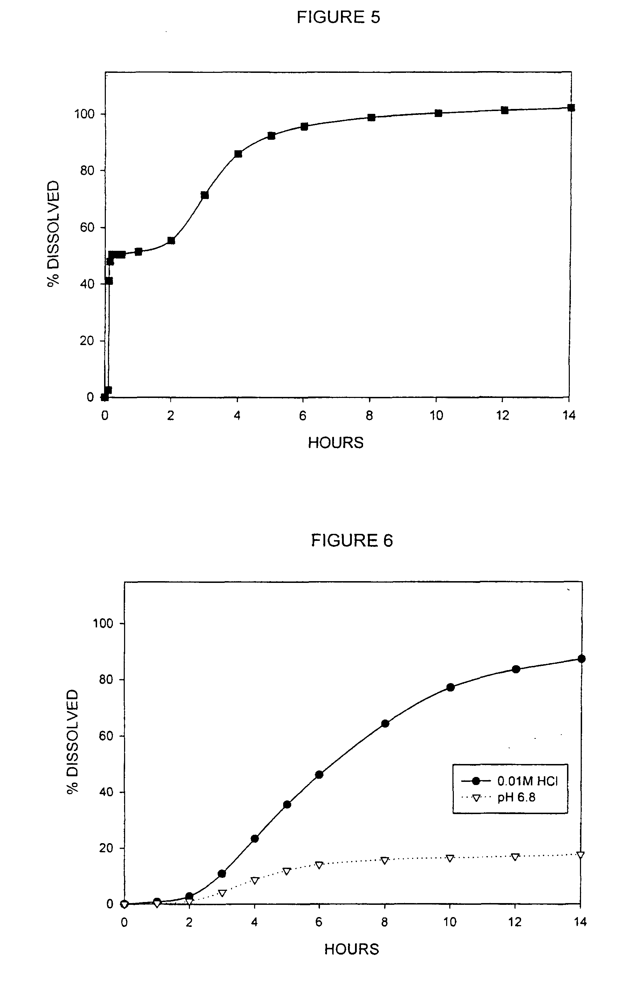

Their dissolution profile in 0.01M hydrochloric acid obtained as described in example 2, is shown in figure 5. The profile parameters in hydrochloric acid are : T₁ = 2.0 h ; T₂ = 5.0 h.

### Comparative example 1: coated pellets containing zolpidem hemitartrate

850 g of pellets coated with zolpidem hemitartrate of example 1 were coated in a GPCG1 fluid bed coater-dryer with the following solution

| | | | |
|---|---|---|---|
| ammonio copolymer Type B ¹ | methacrylate | 11.41 % | 129.6 g |
| ammonio copolymer Type A ² | methacrylate | 0.92 % | 10.5 g |
| triethyl citrate ³ | | 1.37 % | 15.6 g |
| isopropanol | | 51.78 % | 588.0 g |
| acetone | | 34.52 % | 392.0 g |

| | | | |
|---|---|---|---|
| ¹ Eudragit® RS100 commercialised by Röhm Pharma | | | |
| ² Eudragit® RL100 commercialised by Röhm Pharma | | | |
| ³ Eudraflex® commercialised by Röhm Pharma | | | |

After drying, in a ventilated oven at 35°C for 24 hours the dissolution profile of the pellets was measured in 0.01M hydrochloric acid and in pH 6.8, 0.02M phosphate buffer containing 0.1M sodium chloride as described in example 2. The profiles are shown in figure 6. Prolonged dissolution (over about 12 hours) was obtained at 0.01M hydrochloric acid, but the release rate was very slow at pH 6.8.

### Comparative example 2: coated pellets containing zolpidem hemitartate and tartaric acid

735 g of nonpareil beads 16/18 mesh were coated using a suspension with the following composition:

| | | |
|---|---|---|
| tartaric acid | 10 % | 58.4 g |
| povidone K30 ¹ | 2 % | 11.5 g |
| ethanol 95% | 88 % | 505.0 g |

| | | |
|---|---|---|
| ¹ Kollidon® commercialized by BASF | | |

The coated pellets were then coated in a GPG1 fluid bed coater-dryer with the following solution:

| | | |
|---|---|---|
| zolpidem hemitartrate | 11.54 % | 78.125 g |
| povidone K30 ² | 11.54 % | 78.125 g |
| ethanol | 76.92 % | 520.8 g |

| | | |
|---|---|---|
| ² Kollidon® commercialized by BASF | | |

748 g of the zolpidem hemitartrate-tartaric acid-coated beads were then coated with the following solution.

| | | |
|---|---|---|
| ammonio methacrylate copolymer Type B ³ | 11.40 % | 62.5 g |
| ammonio methacrylate copolymer Type A ⁴ | 0.93 % | 5.1 g |
| triethyl citrate ⁵ | 1.37 % | 7.5 g |
| isopropanol | 51.80 % | 283.5 g |
| acetone | 34.50 % | 189.0 g |

| | | |
|---|---|---|
| ³ Eudragit® RS100 commercialised by Röhm Pharma | | |
| ⁴ Eudragit® RL100 commercialised by Röhm Pharma | | |
| ⁵ Eudraflex® commercialised by Röhm Pharma | | |

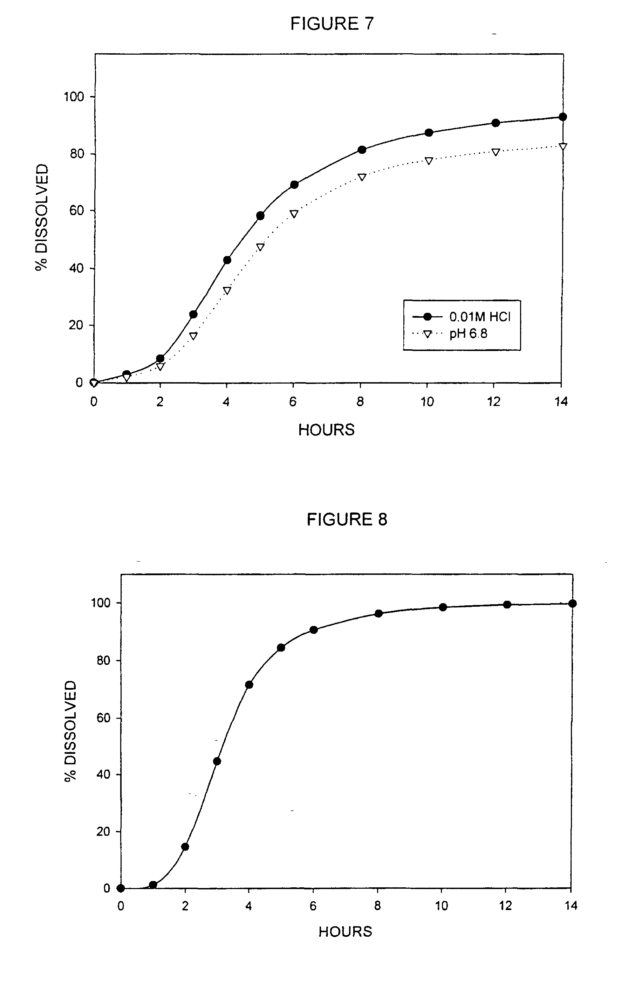

After drying, in a ventilated oven at 35°C for 24 hours the dissolution profile of the pellets were measured in 0.01M hydrochloric acid and in a pH 6.8 0.02M phosphate buffer containing 0.1M sodium chloride as described in example 2. The profiles are shown in figure 7. Dissolution was prolonged and independent of pH.
These two comparative examples show that the delayed release pellets comprising acid present a profile of dissolution independent of the pH and that the addition of a cationic surfactant to the tablet core increases the release rate and extent of release at both acid and neutral pH.

### Example 5: Coated pellets:

Delayed release pellets containing zolpidem tartrate, tartaric acid, and cocamidopropylbetain as an amphoteric surfactant.
1000 g of nonpareil beads 16/18 mesh were coated using a suspension with the following composition, in a GPCG1 fluid bed coater-dryer:

| | | |
|---|---|---|
| tartaric acid | 5.0 % | 78.0 g |
| Cocoamidopropylbetain ¹ | 2.5 % | 39.0 g |
| Povidone VA 64 ² | 5.0 % | 78.0 g |
| Talc | 5.0 % | 78.0 g |
| purified water | 41.25 % | 643.5 g |
| ethanol 95° | 41.25 % | 643.5 g |

| | | |
|---|---|---|
| ¹ Amonyl® 380LC commercialized by Seppic | | |
| ² Kollidon® VA 64 commercialized by BASF | | |

The pellets were then loaded with zolpidem tartrate by coating with the following solution :

| | | |
|---|---|---|
| zolpidem tartrate | 8.3 % | 78 g |
| Povidone VA 64 ³ | 8.3 % | 78 g |
| ethanol 95° | 83.4 % | 784 g |

| | | |
|---|---|---|
| ³ Kollidon® VA 64 commercialized by BASF | | |

Finally 1000 g of the pellets were coated using a polymer solution of the following composition :

| | | | |
|---|---|---|---|
| ammonio copolymer Type B ⁴ | methacrylate | 11.40 % | 83.4 g |
| ammonio copolymer Type A ⁵ | methacrylate | 0.93 % | 6.8 g |
| triethyl citrate ⁶ | | 1.37 % | 10 g |
| isopropanol | | 51.80 % | 379 g |
| acetone | | 34.50 % | 252 g |

| | | | |
|---|---|---|---|
| ⁴ Eudragit® RS100 commercialised by Röhm Pharma | | | |
| ⁵ Eudragit® RL100 commercialised by Röhm Pharma | | | |
| ⁶ Eudraflex® commercialised by Röhm Pharma | | | |

After drying in a ventilated oven, at 30°C for 16 hours the dissolution profile of the pellets in 0.01 M hydrochloric acid was measured, using the method described in the European Pharmacopoeia, with the rotating paddle apparatus, at a stirring speed of 100 rpm. Dissolution medium was 900 ml, 0.01M hydrochloric acid at 37°C ± 0.5°C. The amount of zolpidem dissolved was measured by UV spectrophotometry at 310 nm. The dissolution curve obtained is shown in figure 8.

Example 6 : Tablet containing coated delayed release pellets containing 6 mg zolpidem hemitartrate within a fast-disintegrating matrix containing 6.5 mg zolpidem hemitartrate.

Prolonged release coated pellets were manufactured as described in example 3. The pellets were then spray-coated using the same method with a layer of 20% by mass of microcrystalline cellulose. A granulate of the following composition was then prepared, by wet granulation:

| | |
|---|---|
| zolpidem hemitartrate | 3.0 % |
| lactose | 20.0 % |
| microcrystalline cellulose ¹ | 68.0 % |
| hydroxypropylmethylcellulose 606 | 3.0 % |
| crospovidone ² | 5.0 % |
| magnesium stearate | 1.0 % |

| | |
|---|---|
| ¹ Avicel®, commercialized by FMC | |
| ² Kollidon® CL, commercialised by BASF | |

Pellets and granulate were mixed and compressed into tablets using a rotary press. Each tablet contained 130 mg pellets and 217 mg of the granulate.

## Claims

1. A pharmaceutical composition comprising a short acting hypnotic or a salt thereof **characterised in that** it consists of a timed dual release dosage form adapted to release the short acting hypnotic over a predetermined time period, according to an *in vitro* profile of dissolution when measured in a rotating paddle apparatus of the European pharmacopoeia in 0.01M hydrochloric acid buffer at 37°C, comprising two release pulses, the first release pulse being immediate, having a maximum duration of 30 minutes, and the second release pulse being delayed by a fixed time of between 50 and 200 minutes after the administration, the delayed second release pulse lasting between 30 and 200 minutes.

2. A pharmaceutical composition according to claim 1, **characterised in that** the fixed time is between 60 and 150 minutes.

3. A pharmaceutical composition according to claim 1 or 2, **characterised in that** 40 to 70% of the total amount of the short acting hypnotic is released during the immediate release pulse.

4. A pharmaceutical composition according to any one of claims 1 to 3, **characterised in that** the time for release of 85% of the total amount of the short acting hypnotic is between 2 and 6 hours.

5. A pharmaceutical composition according to any one of claims 1 to 4, **characterised in that** the *in vitro* profile of dissolution is measured in a rotating paddle apparatus of the European pharmacopoeia in 0.01M hydrochloric acid buffer at 37°C, at a stirring speed of 50 or 100 rpm.

6. A pharmaceutical composition comprising a short acting hypnotic or a salt thereof, according to anyone of claims 1 to 5, **characterised in that** it comprises two kinds of pharmaceutical entities: one immediate release entity and one delayed release entity.

7. A pharmaceutical composition according to claim 6, **characterised in that** it consists in a dosage form chosen among capsules, tablets, multilayer tablets, multicoated tablets.

8. A pharmaceutical composition according to claim 6 or 7, **characterised in that** it consists of a capsule comprising one or more immediate release tablets and one or more delayed release tablets.

9. A pharmaceutical composition according to claim 6 or 7, **characterised in that** it consists of a capsule comprising a mixture of delayed release particles and immediate release particles.

10. A pharmaceutical composition according to claim 6 or 7, **characterised in that** it consists of a capsule comprising a mixture of delayed release particles and an immediate release powder.

11. A pharmaceutical composition according to claim 6 or 7, **characterised in that** it consists of a tablet comprising a number of delayed release coated pellets comprising the drug imbedded in a matrix and alternatively **in that**
(i) the matrix comprises the drug,
(ii) immediate release non-coated pellets are mixed to the delayed release coated pellets,
(iii) the delayed coated pellets are further coated with a layer comprising the drug, allowing immediate release form that layer, imbedded in a matrix free from the drug,
(iv) the tablet consists of one or more layers comprising the delayed release pellets imbedded in a matrix free from the drug and one or more layers containing the drug in an immediate release matrix.

12. A pharmaceutical composition according to any one of claims 8 to 11, **characterised in that** the delayed release particles or tablets are coated with a mixture containing at least one ammonio methacrylate copolymer and the core contains a cationic surfactant.

13. A pharmaceutical composition according to any one of claims 8 to 11, **characterised in that** the delayed release particles or tablets are coated with a mixture containing at least one ammonio methacrylate copolymer and the core contains a zwitterionic surfactant.

14. A pharmaceutical composition according to claim 12, **characterised in that** the cationic surfactant is chosen among trimethyl-dimyristoyl-ammonium propionate, dimethyl-dioctadecyl-ammonium bromide, trimethyl-cetyl-ammonium bromide, dimethyl-didodecyl-ammonium bromide, benzalkonium chloride, cetylpyridinium chloride and cetrimide.

15. A pharmaceutical composition according to claim 13, **characterised in that** the zwitterionic surfactants are chosen among N-alkylbetaines, C-alkylbetaines, N-alkylamidobetaines, N-alkylglycines, phosphatidylcholines and lecithines.

16. A pharmaceutical composition according to claim 15, **characterised in that** the zwitterionic surfactant is cocamidopropylbetain.

17. A pharmaceutical composition according to claim 6, **characterised in that** the immediate release entity and the prolonged release entity are administered simultaneously but separately.

18. A pharmaceutical composition according to anyone of claims 6 to 16, **characterised in that** the prolonged release entity comprises a pharmaceutical acceptable organic acid which can be chosen among tartaric, malic, fumaric, lactic, citric, adipic or succinic acid and their acid salts, in the form of racemates or isomers.

19. A pharmaceutical composition according to any one of claims 1 to 18, **characterised in that** the short acting hypnotic belongs to the therapeutic classes of benzodiazepines, cyclopyrrolones, pyrazolopyrimidines, phenotiazines or imidazopyridines.

20. A pharmaceutical composition according to claim 19, **characterised in that** the short acting hypnotic is chosen among triazolam, temazepam, brotizolam, zopiclone, (*R*)-zopiclone, zaleplon, alimemazine, zolpidem and their pharmaceutically acceptable salts thereof.

21. A pharmaceutical composition according to claim 19, **characterised in that** the short acting hypnotic is zolpidem or a pharmaceutically acceptable salt thereof.

22. A pharmaceutical composition according to claim 21, **characterised in that** the salt of zolpidem is zolpidem hemitartrate.

23. A pharmaceutical composition according to anyone of claims 1 to 22, **characterised in that** the composition comprises constituents which, if it is introduced into an optionally alcoholic, aqueous drink, generate visual means on contact with the latter.

24. A pharmaceutical composition according to claim 23, **characterised in that** the visual means are chosen among inclusion of colouring excipients, floating of the composition at the surface of the drink, formation of insoluble particles on the surface of the drink, on the brim of the glass, in the drink and/or on the bottom of the glass or a combination thereof.

## Patentansprüche

1. Pharmazeutische Zubereitung, umfassend ein kurz wirksames Hypnotikum oder ein Salz davon, **dadurch gekennzeichnet, daß** sie aus einer zeitgesteuerten Doppelfreisetzungs-Dosierungsform besteht, die an die Freisetzung des kurz wirksamen Hypnotikums im Verlaufe einer vorbestimmten Zeitdauer angepaßt ist, entsprechend einem *in vitro*-Lösungsprofil, gemessen bei 37°C in einem 0,01M Chlorwasserstoffsäurepuffer in einer Drehflügel-Vorrichtung gemäß der Europäischen Pharmacopoe, umfassend zwei Freisetzungspulse, wobei der erste Freisetzungspuls sofort erfolgt, mit einer Maximaldauer von 30 Minuten, und der zweite Freisetzungspuls um eine festgelegte Zeitdauer von zwischen 50 und 200 Minuten nach der Verabreichung verzögert ist, wobei der verzögerte zweite Freisetzungspuls zwischen 30 und 200 Minuten dauert.

2. Pharmazeutische Zubereitung nach Anspruch 1, **dadurch gekennzeichnet, daß** die festgelegte Zeitdauer zwischen 60 und 150 Minuten beträgt.

3. Pharmazeutische Zubereitung nach Anspruch 1 oder 2, **dadurch gekennzeichnet, daß** 40 bis 70 % der Gesamtmenge des kurz wirksamen Hypnotikums während des sofortigen Freisetzungspulses freigesetzt werden.

4. Pharmazeutische Zubereitung nach einem der Ansprüche 1 bis 3, **dadurch gekennzeichnet, daß** die Zeitdauer zur Freisetzung von 85 % der Gesamtmenge des kurz wirksamen Hypnotikums zwischen 2 und 6 Stunden beträgt.

5. Pharmazeutische Zubereitung nach einem der Ansprüche 1 bis 4, **dadurch gekennzeichnet, daß** das *in vitro*-Lösungsprofil bei 37°C in dem 0.01M Chlorwasserstoffsäurepuffer in einer Drehflügel-Vorrichtung gemäß der Europäischen Pharmacopöe mit einer Rührgeschwindigkeit von 50 oder 100 min⁻¹ gemessen wird.

6. Pharmazeutische Zubereitung, umfassend ein kurz wirksames Hypnotikum oder ein Salz davon gemäß einem der Ansprüche 1 bis 5, **dadurch gekennzeichnet, daß** sie zwei Arten von pharmazeutischen Einheiten enthält, eine Einheit mit sofortiger Freisetzung und eine Einheit mit verzögerter Freisetzung.

7. Pharmazeutische Zubereitung nach Anspruch 6, **dadurch gekennzeichnet, daß** sie in einer Dosierungsform vorliegt, ausgewählt aus Kapseln, Tabletten, Mehrschichttabletten und mehrfach beschichteten Tabletten.

8. Pharmazeutische Zubereitung nach Anspruch 6 oder 7, **dadurch gekennzeichnet, daß** sie aus einer Kapsel besteht, die eine oder mehrere Tabletten mit sofortiger Freisetzung und eine oder mehrere Tabletten mit verzögerter Freisetzung enthält.

9. Pharmazeutische Zubereitung nach Anspruch 6 oder 7, **dadurch gekennzeichnet, daß** sie aus einer Kapsel besteht, die eine Mischung aus Teilchen mit verzögerter Freisetzung und Teilchen mit sofortiger Freisetzung umfaßt.

10. Pharmazeutische Zubereitung nach Anspruch 6 oder 7, **dadurch gekennzeichnet, daß** sie aus einer Kapsel besteht, die eine Mischung aus Teilchen mit verzögerter Freisetzung und ein Pulver mit sofortiger Freisetzung umfaßt:

11. Pharmazeutische Zubereitung nach Anspruch 6 oder 7, **dadurch gekennzeichnet, daß** sie aus einer Tablette besteht, die eine Vielzahl von zur verzögerten Freisetzung beschichteten Pellets umfaßt, bei denen der Wirkstoff in einer Matrix eingebettet ist, und alternativ dadurch, daß
(i) die Matrix den Wirkstoff enthält,
(ii) nichtbeschichtete Pellets mit sofortiger Freisetzung mit den zur verzögerten Freisetzung beschichteten Pellets vermischt sind,
(iii) die beschichteten Pellets mit verzögerter Freisetzung zusätzlich mit einer den Wirkstoff enthaltenden Schicht beschichtet sind, welche die sofortige Freisetzung aus der Schicht ermöglicht, die in der von dem Wirkstoff freien Matrix eingebettet ist,
(iv) die Tablette aus einer oder mehreren Schichten, welche die in einer Wirkstoff-freien Matrix eingebetteten, zur verzögerten Freisetzung beschichteten Pellets enthalten, und einer oder mehreren Schichten, die den Wirkstoff in einer Matrix zur sofortigen Freisetzung enthalten, besteht.

12. Pharmazeutische Zubereitung nach einem der Ansprüche 8 bis 11, **dadurch gekennzeichnet, daß** die Teilchen oder Tabletten mit verzögerter Freisetzung mit einer Mischung beschichtet sind, die mindestens ein Ammoniummethacrylat-Copolymer enthält und der Kern ein kationisches oberflächenaktives Mittel enthält.

13. Pharmazeutische Zubereitung nach einem der Ansprüche 8 bis 11, **dadurch gekennzeichnet, daß** die Teilchen oder Tabletten mit verzögerter Freisetzung mit einer Mischung beschichtet sind, die mindestens ein Ammoniummethacrylat-Copolymer enthält und der Kern ein zwitterionisches oberflächenaktives Mittel enthält.

14. Pharmazeutische Zubereitung nach Anspruch 12, **dadurch gekennzeichnet, daß** das kationische oberflächenaktive Mittel aus Trimethyl-dimyristol-ammoniumpropionat, Dimethyl-dioctadecyl-ammoniumbromid, Trimethyl-cetyl-ammoniumbromid, Dimethyl-didodecyl-ammoniumbromid, Benzalkoniumchlorid, Cetylpyridiniumchlorid und Cetrimid ausgewählt ist.

15. Pharmazeutische Zubereitung nach Anspruch 13, **dadurch gekennzeichnet, daß** die zwitterionischen oberflächenaktiven Mittel ausgewählt sind aus N-Alkylbetainen, C-Alkylbetainen, N-Alkylamidobetainen, N-Alkylglycinen, Phosphatidylcholinen und Lecithinen.

16. Pharmazeutische Zubereitung nach Anspruch 15, **dadurch gekennzeichnet, daß** das zwitterionische oberflächenaktive Mittel Cocamidopropylbetain ist.

17. Pharmazeutische Zubereitung nach Anspruch 6, **dadurch gekennzeichnet, daß** die Einheit mit sofortiger Freisetzung und die Einheit mit verzögerter Freisetzung gleichzeitig, jedoch getrennt verabreicht werden.

18. Pharmazeutische Zubereitung nach einem der Ansprüche 6 bis 16, **dadurch gekennzeichnet, daß** die Einheit mit verzögerter Freisetzung eine pharmazeutisch annehmbare organische Säure enthält, die aus Weinsäure, Äpfelsäure, Fumarsäure, Milchsäure, Citronensäure, Adipinsäure und Succinsäure und deren Salzen ausgewählt ist, in Form von Racematen oder Isomeren.

19. Pharmazeutische Zubereitung nach einem der Ansprüche 1 bis 18, **dadurch gekennzeichnet, daß** das kurz wirksame Hypnotikum zu den therapeutischen Klassen der Benzodiazepine, Cyclopyrrolone, Pyrazolopyrimidine, Phenotiazine oder Imidazopyridine gehört.

20. Pharmazeutische Zubereitung nach Anspruch 19, **dadurch gekennzeichnet, daß** das kurz wirksame Hypnotikum ausgewählt wird aus Triazolam, Temazepam, Brotizolam, Zopiclon, (R)-Zopiclon, Zaleplon, Alimemazin, Zolpidem und deren pharmazeutisch annehmbaren Salzen.

21. Pharmazeutische Zubereitung nach Anspruch 19, **dadurch gekennzeichnet, daß** das kurz wirksame Hypnotikum Zolpidem oder ein pharmazeutisch annehmbares Salz davon ist.

22. Pharmazeutische Zubereitung nach Anspruch 21, **dadurch gekennzeichnet, daß** das Salz von Zolpidem Zolpidem-hemitartrat ist.

23. Pharmazeutische Zubereitung nach einem der Ansprüche 1 bis 22, **dadurch gekennzeichnet, daß** die Zubereitung Bestandteile enthält, die, wenn sie in ein gegebenenfalls alkoholisches, wäßriges Getränk eingebracht wird, im Kontakt damit einen visuellen Effekt erzeugen.

24. Pharmazeutische Zubereitung nach Anspruch 23, **dadurch gekennzeichnet, daß** der visuelle Effekt ausgewählt ist aus dem Einschluß von färbenden Trägermaterialien, dem Aufschwimmen der Zubereitung auf der Oberfläche des Getränks, der Bildung von unlöslichen Teilchen auf der Oberfläche des Getränks, am Rand des Glases, in dem Getränk und/oder auf dem Boden des Glases oder einere Kombination davon.

## Revendications

1. Composition pharmaceutique comprenant un hypnotique à courte durée d'action ou un sel de celui-ci, **caractérisée en ce qu'**elle consiste en une forme posologique à double libération programmée conçue pour libérer l'hypnotique à courte durée d'action sur une durée prédéterminée, selon un profil de dissolution in vitro, mesuré dans un appareil à palette rotative de la Pharmacopée Européenne dans un tampon à l'acide chlorhydrique 0,01M à 37°C, comprenant deux pulses de libération, le premier pulse de libération étant immédiat, ayant une durée maximale de 30 minutes, et le second pulse de libération étant différé d'un temps fixé compris entre 50 et 200 minutes après l'administration, la durée du second pulse de libération différée étant comprise entre 30 et 200 minutes.

2. Composition pharmaceutique selon la revendication 1, **caractérisée en ce que** le temps fixé est compris entre 80 et 150 minutes.

3. Composition pharmaceutique selon la revendication 1 ou 2, **caractérisée en ce que** 40 à 70% de la quantité totale de l'hypnotique à courte durée d'action sont libérés pendant le pulse de libération immédiate.

4. Composition pharmaceutique selon l'une quelconque des revendications 1 à 3, **caractérisée en ce que** le temps qu'il faut pour libérer 85% de la quantité totale de l'hypnotique à courte durée d'action est compris entre 2 et 6 heures.

5. Composition pharmaceutique selon l'une quelconque des revendications 1 à 4, **caractérisée en ce que** le profil de dissolution in vitro est mesuré dans un appareil à palette rotative de la Pharmacopée Européenne dans un tampon à l'acide chlorhydrique 0,01M à 37°C, à une vitesse d'agitation de 50 ou 100 tours/min.

6. Composition pharmaceutique comprenant un hypnotique à courte durée d'action ou un sel de celui-ci, selon l'une quelconque des revendications 1 à 5, **caractérisée en ce qu'**elle comprend deux types d'entités pharmaceutiques: une entité à libération immédiate et une entité à libération différée.

7. Composition pharmaceutique selon la revendication 6, **caractérisée en ce qu'**elle consiste en une forme posologique choisie parmi les gélules, les comprimés, les comprimés multicouches, les comprimés multienrobés.

8. Composition pharmaceutique selon la revendication 6 ou 7, **caractérisée en ce qu'**elle consiste en une gélule comprenant un ou plusieurs comprimés à libération immédiate et un ou plusieurs comprimés à libération différée.

9. Composition pharmaceutique selon la revendication 6 ou 7, **caractérisée en ce qu'**elle consiste en une gélule comprenant un mélange de particules à libération différée et de particules à libération immédiate.

10. Composition pharmaceutique selon la revendication 6 ou 7, **caractérisée en ce qu'**elle consiste en une gélule comprenant un mélange de particules à libération différée et d'une poudre à libération immédiate.

11. Composition pharmaceutique selon la revendication 6 ou 7, **caractérisée en ce qu'**elle consiste en un comprimé comprenant un certain nombre de pellets enrobés à libération différée comprenant le principe actif noyés dans une matrice et, en variante, **en ce que**
(i) la matrice comprend le principe actif,
(ii) les pellets non enrobés à libération immédiate sont mélangés avec les pellets enrobés à libération différée,
(iii) les pellets enrobés à libération différée sont en outre enrobés d'une couche comprenant le principe actif, ce qui permet une libération immédiate de cette couche, noyés dans une matrice exempte du principe actif,
(iv) le comprimé se compose d'une ou de plusieurs couches comprenant les pellets à libération différée dans une matrice exempte du principe actif et d'une ou de plusieurs couches contenant le principe actif dans une matrice à libération immédiate.

12. Composition pharmaceutique selon l'une quelconque des revendications 8 à 11, **caractérisée en ce que** les particules ou comprimés à libération différée sont enrobés d'un mélange contenant au moins un copolymère ammonio-méthacrylate et le noyau contient un tensioactif cationique.

13. Composition pharmaceutique selon l'une quelconque des revendications 8 à 11, **caractérisée en ce que** les particules ou comprimés à libération différée sont enrobés d'un mélange contenant au moins un copolymère ammonio-méthacrylate et le noyau contient un tensioactif zwitterionique.

14. Composition pharmaceutique selon la revendication 12, **caractérisée en ce que** le tensioactif cationique est choisi parmi le propionate de triméthyldimyristoyl-ammonium, le bromure de diméthyl-dioctadécyl-ammonium, le bromure de triméthyl-cétyl-ammonium, le bromure de diméthyl-didodécyl-ammonium, le chlorure de benzalkonium, le chlorure de cétylpyridinium et le cétrimide.

15. Composition pharmaceutique selon la revendication 13, **caractérisée en ce que** les tensioactifs zwitterioniques sont choisis parmi les N-alkylbétaïnes, les C-alkylbétaïnes, les N-alkylamidobétaïnes, les N-alkylglycines, les phosphatidylcholines et les lécithines.

16. Composition pharmaceutique selon la revendication 15, **caractérisée en ce que** le tensioactif zwitterionique est la cocamidopropylbétaïne.

17. Composition pharmaceutique selon la revendication 6, **caractérisée en ce que** l'entité à libération immédiate et l'entité à libération prolongée sont administrées simultanément, mais séparément.

18. Composition pharmaceutique selon l'une quelconque des revendications 6 à 16, **caractérisée en ce que** l'entité à libération prolongée comprend un acide organique pharmaceutiquement acceptable qui peut être choisi parmi l'acide tartrique, malique, fumarique, lactique, citrique, adipique ou succinique, et leurs sels d'acide, sous forme de racémates ou d'isomères.

19. Composition pharmaceutique selon l'une quelconque des revendications 1 à 18, **caractérisée en ce que** l'hypnotique à courte durée d'action appartient aux familles thérapeutiques des benzodiazépines, des cyclopyrrolones, des pyrazolopyrimidines, des phénothiazines ou des imidazopyridines.

20. Composition pharmaceutique selon la revendication 19, **caractérisée en ce que** l'hypnotique à courte durée d'action est choisi parmi le triazolam, le témazépam, le brotizolam, la zopiclone, la (R)-zopiclone, le zaleplon, l'alimémazine, le zolpidem et leurs sels pharmaceutiquement acceptables.

21. Composition pharmaceutique selon la revendication 19, **caractérisée en ce que** l'hypnotique à courte durée d'action est le zolpidem ou un de ses sels pharmaceutiquement acceptables.

22. Composition pharmaceutique selon la revendication 21, **caractérisée en ce que** le sel de zolpidem est l'hémitartrate de zolpidem.

23. Composition pharmaceutique selon l'une quelconque des revendications 1 à 22, **caractérisée en ce que** la composition comprend des constituants qui, si elle est introduite dans une boisson aqueuse, éventuellement alcoolique, engendre des moyens visuels au contact de celle-ci.

24. Composition pharmaceutique selon la revendication 23, **caractérisée en ce que** les moyens visuels sont choisis parmi l'incorporation d'excipients colorants, le flottage de la composition à la surface de la boisson, la formation de particules insolubles sur la surface de la boisson, sur le bord du verre, dans la boisson et/où au fond du verre, ou une combinaison de ceux-ci.
